Europäisches Patentamt
European Patent Office
Office européen des brevets

(19)

(11) Veröffentlichungsnummer: **0 077 963**
**A1**

(12) # EUROPÄISCHE PATENTANMELDUNG

(21) Anmeldenummer: 82109385.3

(22) Anmeldetag: 11.10.82

(51) Int. Cl.³: C 07 C 79/355
C 07 C 76/02, A 01 N 33/22

(30) Priorität: 22.10.81 DE 3141859

(43) Veröffentlichungstag der Anmeldung:
04.05.83 Patentblatt 83/18

(84) Benannte Vertragsstaaten:
AT BE CH DE FR GB IT LI NL

(71) Anmelder: BAYER AG
Zentralbereich Patente, Marken und Lizenzen
D-5090 Leverkusen 1, Bayerwerk(DE)

(72) Erfinder: Förster, Heinz, Dr.
Am Eckbusch 47
D-5600 Wuppertal 1(DE)

(72) Erfinder: Eue, Ludwig, Dr.
Paul-Klee-Strasse 36
D-5090 Leverkusen 1(DE)

(72) Erfinder: Schmidt, Robert, Dr.
Im Waldwinkel 110
D-5060 Bergisch-Gladbach 2(DE)

(72) Erfinder: Lürssen, Klaus, Dr.
August-Kierspel-Strasse 89
D-5060 Bergisch-Gladbach 2(DE)

(54) **2-Nitro-5-(4-trifluormethyl-phenoxy)-benzylderivate, Verfahren zu ihrer Herstellung sowie ihre Verwendung als Herbizide und Pflanzenwuchsregulatoren.**

(57) Neue 2-nitro-5-(4-trifluormethyl-phenoxy)-benzylderivate der Formel

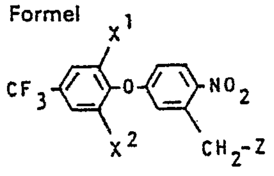

(I)

in welcher

$X^1$ und $X^2$ unabhängig voneinander für Wasserstoff oder Chlor stehen und

Z für Fluor, Chlor, Brom, Jod oder für den Rest–O–R steht, worin, R für Methyl, Ethyl, Propyl, Butyl, Trifluormethyl, 2,2,2-Trifluorethyl, 2-Chlorethyl, 2-Methoxyethyl, 2-Ethoxyethyl, 2-Propoxyethyl, Methoxycarbonylmethyl, Methoxycarbonylethyl, Ethoxycarbonylmethyl, Ethoxycarbonylethyl, Dimethylaminocarbonylmethyl, Dimethylaminocarbonylethyl, Diethylaminocarbonylmethyl, Diethylaminocarbonylethyl, Methylpiperidinocarbonylmethyl, Methylpiperidinocarbonylethyl, Ethylpiperidinocarbonylmethyl, N-Methyl-N-phenyl-aminocarbonylmethyl oder N-methyl-N-phenyl-aminocarbonylethyl steht,

mehrere Verfahren zu deren Herstellung sowie die Verwendung der neuen Stoffe zur Unkrautbekämpfung und zur Regulierung des Pflanzenwachstums.

EP 0 077 963 A1

BAYER AKTIENGESELLSCHAFT 5090 Leverkusen, Bayerwerk

Zentralbereich
Patente, Marken und Lizenzen Dü-by-c
Ib

# 2-Nitro-5-(4-trifluormethyl-phenoxy)-benzylderivate, Verfahren zu ihrer Herstellung sowie ihre Verwendung als Herbizide und Pflanzenwuchsregulatoren

Die Erfindung betrifft neue 2-Nitro-5-(4-trifluormethyl-phenoxy)-benzylderivate, mehrere Verfahren zu deren Herstellung sowie deren Verwendung als Herbizide und Pflanzenwuchsregulatoren.

Es ist bereits bekannt geworden, daß bestimmte Diphenylether herbizide Eigenschaften aufweisen (vgl. US-PS 3 928 416). So läßt sich zum Beispiel 2-Nitro-5-(2,6-dichlor-4-trifluormethyl-phenoxy)-phenol zur Bekämpfung von Unkraut verwenden. Die herbizide Wirkung dieses Stoffes ist gut, jedoch ist die Selektivität nicht immer ausreichend.

Es wurden nun neue 2-Nitro-5-(4-trifluormethyl-phenoxy)-benzylderivate der Formel

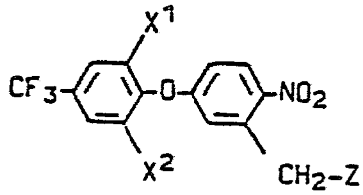

(I)

in welcher

$X^1$ und $X^2$ unabhängig voneinander für Wasserstoff oder Chlor stehen und

Z für Fluor, Chlor, Brom, Jod oder für den Rest -O-R steht, worin

R für Methyl, Ethyl, Propyl, Butyl, Trifluormethyl, 2,2,2-Trifluorethyl, 2-Chlorethyl, 2-Methoxyethyl, 2-Ethoxyethyl, 2-Propoxyethyl, Methoxycarbonylmethyl, Methoxycarbonylethyl, Ethoxycarbonylmethyl, Ethoxycarbonylethyl, Dimethylaminocarbonylmethyl, Dimethylaminocarbonylethyl, Diethylaminocarbonylmethyl, Diethylaminocarbonylethyl, Methylpiperidinocarbonylmethyl, Methylpiperidinocarbonylethyl, Ethylpiperidinocarbonylmethyl, N-Methyl-N-phenylaminocarbonylmethyl oder N-Methyl-N-phenyl-aminocarbonylethyl steht,

gefunden.

Weiterhin wurde gefunden, daß man die neuen Verbindungen der Formel (I) erhält, wenn man

a) 2-Nitro-5-(4-trifluormethyl-phenoxy)-benzylalkohole der Formel

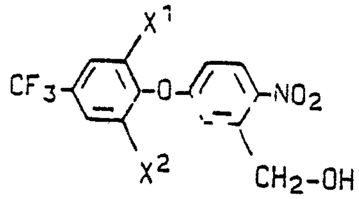

(II)

in welcher

$X^1$ und $X^2$ die oben angegebene Bedeutung haben,

α) mit zum Austausch von Hydroxy gegen Chlor oder Brom geeigneten Stoffen, gegebenenfalls in Gegenwart eines Katalysators und gegebenenfalls in Gegenwart eines Verdünnungsmittels umsetzt,

oder

ß) mit Alkylierungsmitteln der Formel

$$Z^1 - R \qquad (III)$$

in welcher

$Z^1$ für Chlor, Brom oder Jod steht und

R die oben angegebene Bedeutung hat,

gegebenenfalls in Gegenwart eines Säureakzeptors und gegebenenfalls in Gegenwart eines Verdünnungsmittels umsetzt,

oder

γ) mit Dimethylsulfat oder Diethylsulfat gegebenenfalls in Gegenwart eines Säureakzeptors und gegebenenfalls in Gegenwart eines Verdünnungsmittels umsetzt,

oder

b) 2-Nitro-5-(4-trifluormethyl-phenoxy)-benzylhalogenide der Formel

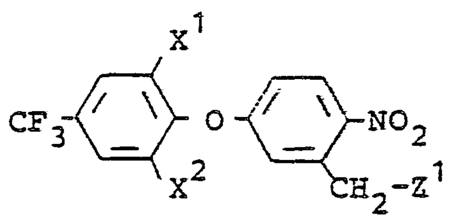

(Ia)

in welcher

$X^1$, $X^2$ und $Z^1$ die oben angegebene Bedeutung haben,

α) mit Fluorierungsmitteln oder, sofern $Z^1$ nicht für Jod steht, mit Alkaliiodiden gegebenenfalls in Gegenwart eines Katalysators sowie gegebenenfalls in Gegenwart eines Verdünnungsmittels umsetzt,

oder

β) mit Verbindungen der Formel

$$R - O - X \qquad (IV)$$

in welcher

R die oben angegebene Bedeutung hat und

X für Wasserstoff, Lithium, Natrium oder Kalium steht,

gegebenenfalls in Gegenwart eines Säureakzeptors sowie gegebenenfalls in Gegenwart eines Verdünnungsmittels umsetzt,

oder

c) 3-(4-Trifluormethyl-phenoxy)-benzylderivate der Formel

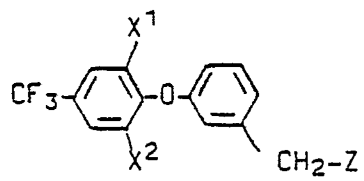

(V)

in welcher

$X^1$,$X^2$ und Z die oben angegebene Bedeutung haben,

mit Salpetersäure gegebenenfalls in Gegenwart eines Katalysators und gegebenenfalls in Gegenwart eines Verdünnungsmittels umsetzt.

Schließlich wurde gefunden, daß sich die neuen 2-Nitro-5-(4-trifluormethyl-phenoxy)-benzylderivate der Formel (I) durch hervorragende herbizide und pflanzenwuchsregulierende Eigenschaften auszeichnen.

Überraschenderweise besitzen die erfindungsgemäßen 2-Nitro-5-(4-trifluormethyl-phenoxy)-benzylderivate der Formel (I) eine wesentlich bessere selektive herbizide Wirksamkeit als aus dem Stand der Technik bekannte Stoffe ähnlicher Konstitution und Wirkungsrichtung. So übertreffen die erfindungsgemäßen Stoffe zum Beispiel das vorbekannte 2-Nitro-(2,6-dichlor-4-trifluormethyl-phenoxy)-phenol bezüglich der selektiven herbiziden Eigenschaften. Darüber hinaus lassen sich die erfindungsgemäßen Verbindungen auch überraschenderweise zur Regulierung des Pflanzenwachstums einsetzen.

Eine bevorzugte Gruppe von erfindungsgemäßen Verbindungen sind diejenigen Stoffe der Formel (I), in denen

$X^1$ für Wasserstoff oder Chlor steht,

$X^2$ für Wasserstoff oder Chlor steht und

Z für Fluor, Chlor, Brom, Jod oder für den Rest -OR steht, worin R vorzugsweise für Methyl, Ethyl, n-Propyl, Isopropyl, n-Butyl, iso-Butyl, sek.-Butyl oder tert.-Butyl steht.

Bevorzugte erfindungsgemäße Verbindungen sind auch solche Stoffe der Formel (I), in denen

$X^1$ für Wasserstoff oder Chlor steht,

$X^2$ für Wasserstoff oder Chlor steht und

Z für den Rest OR steht, worin R für Trifluormethyl, 2,2,2-Trifluorethyl, 2-Chlorethyl, 2-Methoxyethyl, 2-Ethoxyethyl, 2-n-Propoxyethyl, 2-Isopropoxyethyl, Methoxycarbonylmethyl, Methoxycarbonylethyl, Ethoxycarbonylmethyl oder Ethoxycarbonylethyl steht.

Bevorzugte erfindungsgemäße Verbindungen sind schließlich auch solche Stoffe der Formel (I), in denen

$X^1$ für Wasserstoff oder Chlor steht,

$X^2$ für Wasserstoff oder Chlor steht und

Z für den Rest OR steht, worin R für Dimethylaminocarbonylmethyl, Dimethylaminocarbonylethyl, Diethylaminocarbonylmethyl, Diethylaminocarbonylethyl, Methylpiperidinocarbonylmethyl, Methylpiperidinocarbonylethyl, Ethylpiperidinocarbonylmethyl, N-Methyl-N-phenyl-aminocarbonylmethyl oder N-Methyl-N-phenyl-aminocarbonylethyl steht.

Als Beispiele für 2-Nitro-5-(4-trifluormethyl-phenoxy)-benzylderivate der Formel (I) seien die in der folgenden Tabelle aufgeführten Stoffe genannt:

Tabelle 1

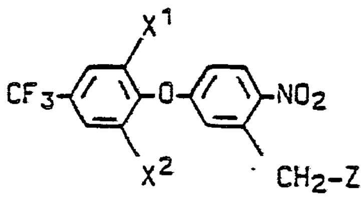

(I)

| $X^1$ | $X^2$ | Z |
|---|---|---|
| Cl | Cl | $OC_3H_7$-iso |
| H | Cl | $OC_3H_7$-n |
| H | Cl | $O-CH_2-CF_3$ |
| H | Cl | $O-CH_2-CH_2-Cl$ |
| Cl | Cl | $O-CH_2-CH_2-OCH_3$ |
| H | Cl | $OC_2H_5$ |
| Cl | Cl | $OC_2H_5$ |
| H | Cl | $OC_3H_7$-iso |
| Cl | Cl | $OC_3H_7$-n |
| Cl | Cl | $O-CH_2-CF_3$ |
| Cl | Cl | $O-CH_2-CH_2-Cl$ |
| H | Cl | $O-CH_2-CH_2-OCH_3$ |

Tabelle 1 (Fortsetzung)

| $X^1$ | $X^2$ | Z |
|---|---|---|
| H | Cl | $O-CH_2-CO-OCH_3$ |
| Cl | Cl | $O-CH_2-CO-OCH_3$ |
| H | Cl | $O-CH_2-CO-OC_2H_5$ |
| Cl | Cl | $O-CH_2-CO-OC_2H_5$ |
| H | Cl | $O-CH_2-CO-N(CH_3)_2$ |
| Cl | Cl | $O-CH_2-CO-N(CH_3)_2$ |
| H | Cl | $O-CH_2-CO-N(C_2H_5)_2$ |
| Cl | Cl | $O-CH_2-CO-N(C_2H_5)_2$ |
| H | Cl | $O-CH_2-CO-$ 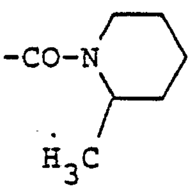 |
| Cl | Cl | $O-CH_2-CO-$ 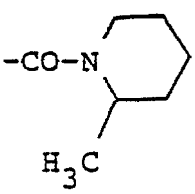 |
| H | Cl | $O-CH_2-CO-$ 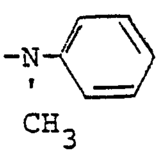 |

Tabelle 1 (Fortsetzung)

| $X^1$ | $X^2$ | Z |
|---|---|---|
| Cl | Cl | 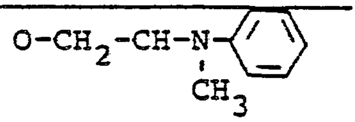 |
| Cl | Cl | I |
| H | Cl | I |
| H | Cl | F |
| Cl | Cl | F |
| H | H | $OCH_3$ |
| H | H | I |
| H | Cl | $OCH-COO-CH_3$<br>$\,\vert$<br>$CH_3$ |
| H | Cl | $OCH-COO-C_2H_5$<br>$\,\vert$<br>$CH_3$ |
| Cl | Cl | $OCH-COO-C_2H_5$<br>$\,\vert$<br>$CH_3$ |
| H | Cl | $OCH-CO-N(C_2H_5)_2$<br>$\,\vert$<br>$CH_3$ |
| Cl | Cl | $OCH-CO-N(C_2H_5)_2$<br>$\,\vert$<br>$CH_3$ |

Verwendet man 2-Nitro-5-(2-chlor-4-trifluormethyl-phenoxy)-benzylalkohol und Thionylchlorid als Ausgangsstoffe, so kann der Verlauf des erfindungsgemäßen Verfahrens (a), Variante α, durch das folgende Formelschema wiedergegeben werden:

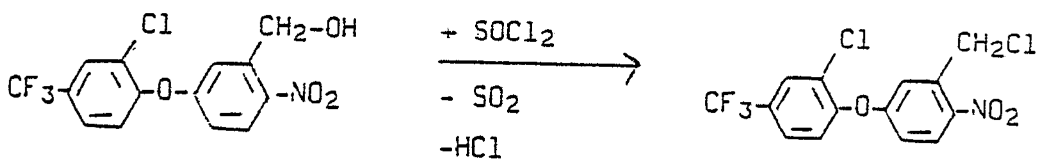


Verwendet man 2-Nitro-5-(2-chlor-4-trifluormethyl-phenoxy)-benzylalkohol und n-Propylbromid als Ausgangsstoffe, so kann der Verlauf des erfindungsgemäßen Verfahrens (a), Variante ß, durch das folgende Formelschema wiedergegeben werden:

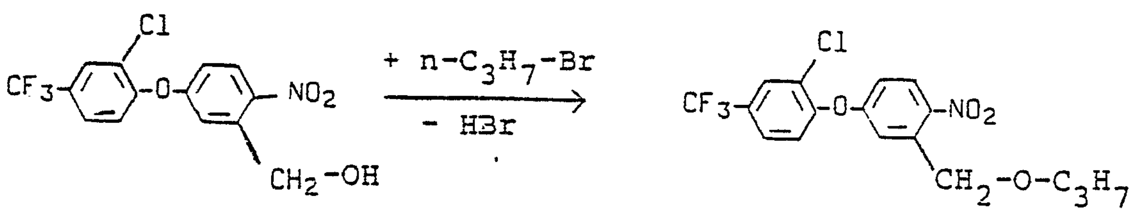


Verwendet man 2-Nitro-5-(2,6-dichlor-4-trifluormethyl-phenoxy)-benzylalkohol und Diethylsulfat aus Ausgangsstoffe, so kann der Verlauf des erfindungsgemäßen Verfahrens (a), Variante γ, durch das folgende Formelschema wiedergegeben werden:

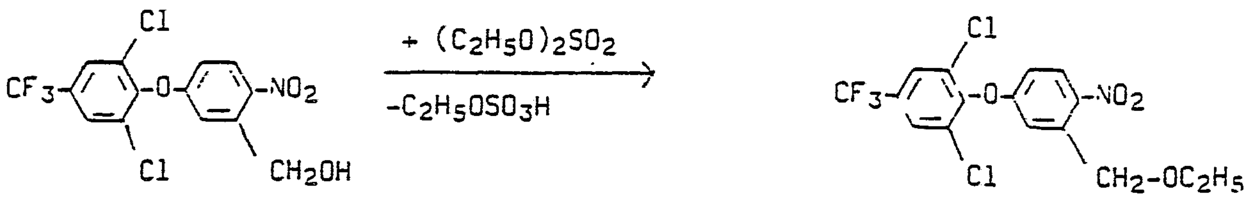


Verwendet man 2-Nitro-5-(2-chlor-4-trifluormethyl-phenoxy)-benzylchlorid und Kaliumjodid als Ausgangsstoffe, so kann der Verlauf des erfindungsgemäßen Verfahrens (b), Variante α, durch folgendes Formelschema wiedergegeben werden:

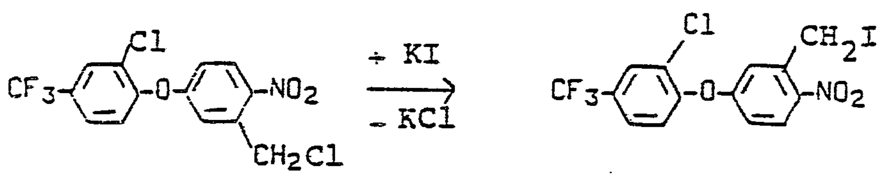


Verwendet man 2-Nitro-5-(2,6-dichlor-4-trifluormethyl-phenoxy)-benzylchlorid und Natriummethylat aus Ausgangsstoffe, so kann der Verlauf des erfindungsgemäßen Verfahrens (b), Variante ß, durch das folgende Formelschema wiedergegeben werden:

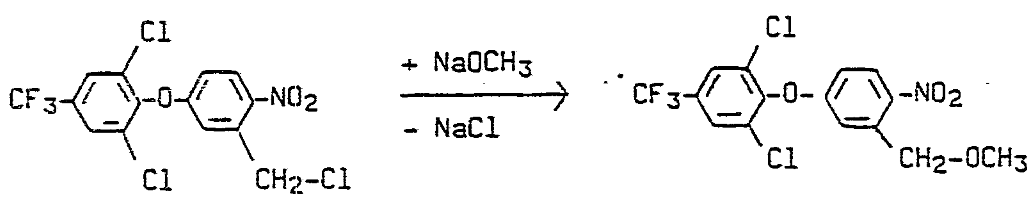


Verwendet man 3-(2,6-Dichlor-4-trifluormethyl-phenoxy)-benzyl-methylether als Ausgangsstoff und Salpetersäure als Nitrierungsmittel, so kann der Verlauf des erfindungsgemäßen Verfahrens (c) durch das folgende Formelschema wiedergegeben werden:

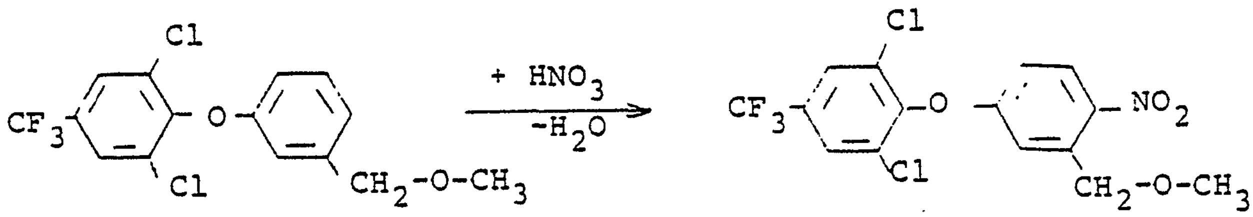


Die bei der Durchführung des erfindungsgemäßen Verfahrens (a) als Ausgangsstoffe benötigten 2-Nitro-5-(4-trifluormethyl-phenoxy)-benzylalkohole der Formel (II) sind bisher noch nicht bekannt.

Man erhält diese Verbindungen wenn man 2-Nitro-5-(4-trifluormethyl-phenoxy)-benzoesäurechloride der Formel

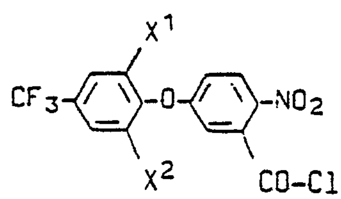

(VI)

in welcher

$X^1$ und $X^2$ die oben angegebene Bedeutung haben,

mit Hydridkomplexen, wie z.B. Natriumboranat, in Gegenwart von Verdünnungsmitteln, wie z.B. Diglycoldimethylether (Diglyme) bei Temperaturen zwischen -20 und +20°C umsetzt.

Die Aufarbeitung kann wie üblich durchgeführt werden, beispielsweise durch Verdünnen mit Wasser, Ansäuern, z.B. mit Essigsäure, und Isolierung der kristallin anfallenden Produkte der Formel (II) durch Filtration.

0077963

Die 2-Nitro-5-(4-trifluormethyl-phenoxy)-benzoesäure-chloride der Formel (VI) sind bereits bekannt (vgl. DE-OS 2 950 401).

Man erhält die Ausgangsstoffe der Formel (II) auch, wenn man 2-Nitro-5-(4-trifluormethyl-phenoxy)-benzaldehyde der Formel

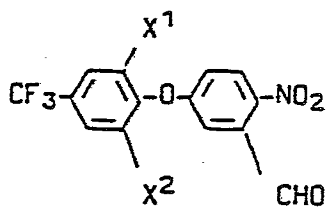

(VII)

in welcher

$X^1$ und $X^2$ die oben angegebene Bedeutung haben,

mit Hydridkomplexen, wie z.B. Natriumboranat, wie oben angegeben umsetzt.

Die 2-Nitro-5-(4-trifluormethyl-phenoxy)-benzaldehyde der Formel (VII) sind ebenfalls bereits bekannt (vgl. DE-OS 3 017 795).

Als Stoffe, die zum Austausch von Hydroxy gegen Chlor oder Brom geeignet sind und beim erfindungsgemäßen Verfahren (a), Variante $\alpha$, als Reaktionskomponenten eingesetzt werden können, seien genannt: Thionylchlorid, Phosphortrichlorid, Phosphorpentachlorid, Phosphortribromid, Chlorwasserstoff und Bromwasserstoff.

Als Katalysatoren kommen bei der Durchführung des erfindungsgemäßen Verfahrens (a), Variante $\alpha$, alle für derartige Halogenierungen üblichen Reaktionsbeschleuniger in Frage. Als Beispiele genannt seien Schwefelsäure, p-Toluolsulfonsäure, Dimethylformamid und Zinkchlorid.

Als Verdünnungsmittel kommen bei dem erfindungsgemäßen Verfahren (a), Variante $\alpha$, praktisch alle unter den Reaktionsbedingungen inerten organischen Lösungsmittel in Betracht. Hierzu gehören insbesondere aliphatische und aromatische, gegebenenfalls halogenierte Kohlenwasserstoffe, wie Pentan, Heptan, Hexan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol sowie Ether, wie Diethyl-, Dipropyl- und Dibutylether, Glycoldimethylether und Diglycoldimethylether, Tetrahydrofuran und Dioxan.

Die Reaktionstemperaturen können bei der Durchführung des erfindungsgemäßen Verfahrens (a), Variante $\alpha$, innerhalb eines größeren Bereichs variiert werden. Im allgemeinen arbeitet man bei Temperaturen zwischen 0 und 150°C, vorzugsweise zwischen 10 und 100°C.

Das erfindungsgemäße Verfahren (a), Variante $\alpha$, wird im allgemeinen bei Normaldruck durchgeführt.

Zur Durchführung des erfindungsgemäßen Verfahrens (a), Variante α, setzt man auf 1 Mol 2-Nitro-5-(4-trifluormethyl-phenoxy)-benzylalkohol der Formel (II) im allgemeinen 1 bis 2 Mol, vorzugsweise 1 bis 1,5 Mol Halogenierungsmittel ein. Die Reaktionskomponenten werden bei Raumtemperatur gegebenenfalls unter Zugabe eines Verdünnungsmittels und eines Katalysators vermischt und gegebenenfalls bei erhöhter Temperatur bis zum Ende der Umsetzung gerührt. Die Aufarbeitung erfolgt nach üblichen Methoden.

In einer bevorzugten Aufarbeitungsweise wird das Reaktionsgemisch mit Wasser verdünnt. Soweit hierbei das Produkt der Formel (I) kristallin anfällt, wird es durch Absaugen isoliert. Fällt das Produkt in öliger Form an, wird es mit einem mit Wasser praktisch nicht mischbaren organischen Lösungsmittel, wie z.B. Cyclohexan extrahiert. Die Extraktionslösung wird mit Wasser neutral gewaschen, getrocknet, filtriert und eingeengt. Nach sorgfältigem Abziehen der flüchtigen Bestandteile bleibt das gewünschte Produkt zurück. Es wird durch seinen Brechungsindex charakterisiert.

Die bei dem erfindungsgemäßen Verfahren (a), Variante ß, als Reaktionskomponenten benötigten Alkylierungsmittel sind durch die Formel (III) definiert. In dieser Formel haben $Z^1$ und R diejenigen Bedeutungen, die bereits im Zusammenhang mit der Beschreibung der erfindungsgemäßen Stoffe der Formel (I) für diese Reste genannt wurden.

Als Beispiele für Alkylierungsmittel der Formel (III) seien genant: Methylchlorid, Ethylchlorid, Methylbromid, Ethylbromid, Methyliodid, Bromessigsäuremethylester und α-Brompropionsäuremethylester.

Bei der Durchführung des erfindungsgemäßen Verfahrens (a), Varianten ß und γ, arbeitet man vorzugsweise in Gegenwart eines Säureakzeptors. Als solche können alle üblichen Säurebindemittel Verwendung finden. Vorzugsweise in Frage kommen Alkali-hydroxide, wie z.B. Natrium- und Kaliumhydroxid, Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin und Pyridin.

Als Verdünnungsmittel können bei der Durchführung des erfindungsgemäßen Verfahrens (a), Varianten ß und γ, praktisch alle inerten organischen Solventien verwendet werden. Vorzugsweise in Betracht kommen aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethylether und Dibutylether, Glycoldimethylether und Diglycoldimethylether, Tetrahydrofuran und Dioxan, Ketone, wie Aceton, Methylethyl-, Methyl-isopropyl- und

Methyl-isobutylketon, Ester wie Essigsäure-methylester und -ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Die Reaktionstemperaturen können bei der Durchführung des erfindungsgemäßen Verfahrens (a), Varianten ß und γ, innerhalb eines größeren Bereichs variiert werden. Im allgemeinen arbeitet man bei Temperaturen zwischen 0 und 150°C, vorzugsweise zwischen 10 und 100°C.

Das erfindungsgemäße Verfahren (a), Varianten ß und γ, wird im allgemeinen bei Normaldruck oder bei erhöhtem Druck bis etwa 100 bar durchgeführt.

Zur Durchführung des erfindungsgemäßen Verfahrens (a), Varianten ß und γ, setzt man auf 1 Mol 2-Nitro-5-(4-trifluormethyl-phenoxy)-benzylalkohol der Formel (II) im allgemeinen 1 bis 3 Mol, vorzugsweise 1,1 bis 2 Mol Alkylierungsmittel der Formel (III) bzw. Dimethylsulfat oder Diethylsulfat ein. Die Reaktionskomponenten werden bei Raumtemperatur gegebenenfalls unter Zugabe eines Säureakzeptors und eines Verdünnungsmittels vermischt und gegebenenfalls bei erhöhter Temperatur bis zum Ende der Umsetzung gerührt. Die Aufarbeitung erfolgt nach üblichen Methoden. Im allgemeinen wird sie in der Weise durchgeführt wie es oben bereits für das Verfahren (a), Variante α, beschrieben wurde.

Die bei dem erfindungsgemäßen Verfahren (b) als Ausgangsstoffe benötigten 2-Nitro-5-(4-trifluormethyl-phenoxy)-benzylhalogenide sind durch die Formel (Ia) definiert. Diese Verbindungen sind bisher noch nicht bekannt; sie lassen sich jedoch nach dem erfindungsgemäßen Verfahren (a), Variante α, in einfacher Weise herstellen.

Als Fluorierungsmittel können beim erfindungsgemäßen Verfahren (b), Variante α, Verbindungen eingesetzt werden, welche zur Einführung von Fluor an Stelle anderer Halogene geeignet sind. Als Beispiele für solche Fluorierungsmittel seien genannt: Kaliumfluorid, Silberfluorid, Quecksilber(I)fluorid, Antimon(III)fluorid und Fluorwasserstoff.

Als Katalysator, welcher hierbei gegebenenfalls zu verwenden ist, sei Antimon(V)chlorid genannt.

Als Alkaliiodide, welche bei Verfahren (b), Variante α, eingesetzt werden können, seien Natriumiodid und Kaliumiodid genannt.

Als Verdünnungsmittel kommen bei dem erfindungsgemäßen Verfahren (b), Variante α, die oben bei der Beschreibung von Verfahren (a), Varianten ß und γ, angegebenen Solventien in Betracht.

Die Reaktionstemperaturen können beim erfindungsgemäßen Verfahren (b), Variante $\alpha$, innerhalb eines größeren Bereichs variiert werden. Im allgemeinen arbeitet man bei Temperaturen zwischen 0 und 200°C, vorzugsweise zwischen 10 und 150°C.

Das erfindungsgemäße Verfahren (b), Variante $\alpha$, wird im allgemeinen bei Normaldruck oder bei erhöhtem Druck bis etwa 100 bar durchgeführt.

Zur Durchführung des erfindungsgemäßen Verfahrens (b), Variante $\alpha$, setzt man auf 1 Mol 2-Nitro-5-(4-trifluormethyl-phenoxy)-benzylhalogenid der Formel (Ia) im allgemeinen 1 bis 2 Mol, vorzugsweise 1 bis 1,5 Mol Fluorierungsmittel bzw. Alkaliiodid, ein. Die Reaktionskomponenten werden bei Raumtemperatur gegebenenfalls unter Zugabe eines Katalysators vermischt und gegebenenfalls bei erhöhter Temperatur bis zum Ende der Umsetzung gerührt. Die Aufarbeitung erfolgt nach üblichen Methoden. Im allgemeinen wird sie in der Weise durchgeführt wie es oben bereits für das erfindungsgemäße Verfahren (a), Variante $\alpha$, beschrieben wurde.

Die bei dem erfindungsgemäßen Verfahren (b), Variante ß, als Reaktionskomponenten benötigten Verbindungen sind durch die Formel (IV) definiert. In dieser Formel haben R und X diejenigen Bedeutungen, die oben bereits für diese Reste genannt wurden.

Als Beispiele für Verbindungen der Formel (IV) seien genannt:
Methanol und Lithium-, Natrium- und Kaliummethylat, sowie Ethanol und Lithium-, Natrium- und Kaliumethylat oder Hydroxyessigsäuremethyl- oder -ethylester oder Milchsäureethylester oder -methylester.

Das erfindungsgemäße Verfahren (b), Variante ß, wird vorzugsweise in Gegenwart von Verdünnungsmitteln durchgeführt. Als Verdünnungsmittel kommen hierbei vorzugsweise diejenigen Solventien in Betracht, welche bereits im Zusammenhang mit der Beschreibung von Verfahren (a), Varianten ß und γ, genannt wurden. Weiter können auch die Alkohole der Formel (IV) bei Einsatz im Überschuß als Verdünnungsmittel dienen.

Als Säureakzeptoren, die bei der Durchführung des erfindungsgemäßen Verfahrens b, Variante ß, eingesetzt werden können, kommen vorzugsweise diejenigen Säurebindemittel in Betracht, die bereits im Zusammenhang mit der Beschreibung des Verfahrens a, Varianten ß und γ, vorzugsweise genannt wurden.

Die Reaktionstemperaturen können auch beim erfindungsgemäßen Verfahren (b), Variante ß, in einem größeren Bereich variiert werden. Im allgemeinen arbeitet man bei Temperaturen zwischen 0 und 150°C, vorzugsweise zwischen 10 und 100°C.

Das erfindungsgemäße Verfahren (b), Variante ß, wird im allgemeinen bei Normaldruck oder bei erhöhtem Druck bis etwa 10 bar durchgeführt.

Zur Durchführung des erfindungsgemäßen Verfahrens (b), Variante ß, setzt man auf 1 Mol 2-Nitro-5-(4-trifluormethyl-phenoxy)-benzylhalogenid der Formel (Ia) im allgemeinen 1 bis 2, vorzugsweise 1,1 bis 1,5 Mol einer Verbindung der Formel (IV) ein. Die Reaktionskomponenten werden bei Raumtemperatur gegebenenfalls unter Zugabe eines Säureakzeptors und eines Verdünnungsmittels vermischt und gegebenenfalls bei erhöhter Temperatur bis zum Ende der Umsetzung gerührt. Die Aufarbeitung erfolgt nach üblichen Methoden. Im allgemeinen wird sie so durchgeführt wie es oben bereits für das erfindungsgemäße Verfahren (a), Variante α, beschrieben wurde.

Das erfindungsgemäße Verfahren (c) wird gegebenenfalls in Gegenwart eines Katalysators durchgeführt. Als Katalysatoren kommen vorzugsweise Protonensäuren, wie z.B. Schwefelsäure oder Essigsäure, in Betracht.

Die Reaktionstemperatur wird bei Verfahren (c) im allgemeinen zwischen -20 und +80°C, vorzugsweise zwischen 0 und 50°C gehalten. Verfahren (c) wird im allgemeinen bei Normaldruck durchgeführt.

Zur Durchführung des erfindungsgemäßen Verfahrens (c) setzt man auf 1 Mol 5-(4-Trifluormethyl-phenoxy)-benzyl-Derivat der Formel (V) im allgemeinen 0,9 bis 2 Mol, vorzugsweise 1,0 bis 1,3 Mol Salpetersäure und gegebenenfalls etwa die gleiche Menge eines Katalysators ein. Die Ausgangskomponenten werden vorzugsweise unter Eiskühlung zusammengegeben und dann gegebenenfalls bei leicht erhöhter Temperatur bis zum Ende der Umsetzung gerührt.

Die Aufarbeitung erfolgt nach üblichen Methoden, beispielsweise durch Eingießen des Reaktionsgemisches in Eiswasser, Absaugen und gegebenenfalls Umkristallisieren.

Es ist auch möglich, so aufzuarbeiten, wie es oben für Verfahren (a), Variante $\alpha$, beschrieben wurde.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe sind besonders gut zur selektiven Unkrautbekämpfung in verschiedenen wichtigen Kulturen, wie zum Beispiel Mais und Baumwolle, geeignet. Darüber hinaus können sie auch sehr gut zur Wuchshemmung, sowie zur Entlaubung und Austrocknung der Blätter bei Baumwolle eingesetzt werden.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe, Feinstverkapselungen in polymeren Stoffen.
Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder

Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silicate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische

Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden oder Pflanzenwachstumsregulatoren zur Unkrautbekämpfung bzw. als Pflanzenwuchsregulatoren Verwendung finden, wobei Fertigformulierungen oder Tankmischungen möglich sind. Auch eine Mischung mit anderen bekannten Wirkstoffen, wie Fungiziden, Insektiziden, Akariziden,

Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können bei einer Anwendung als Herbizide sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Für die Anwendung der erfindungsgemäßen Wirkstoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Die angewandte Wirkstoffmenge kann in einem größeren Bereich schwanken. Sie hängt im wesentlichen ab von der Art des gewünschten Effektes. Im allgemeinen liegen

die Aufwandmengen zwischen 0,01 und 50 kg Wirkstoff pro Hektar Bodenfläche, vorzugsweise zwischen 0,05 und 10 kg pro ha.

Die Herstellung und die Verwendung der erfindungsgemäßen Wirkstoffe gehen aus den nachfolgenden Beispielen hervor.

Herstellungsbeispiele

Beispiel 1

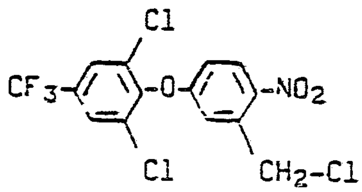

14,3 g (0,12 Mol) Thionylchlorid werden bei 20°C zu einer Mischung aus 38,2 g (0,1 Mol) 2-Nitro-5-(2,6-dichlor-4-trifluormethyl-phenoxy)-benzylalkohol, 80 ml Tetrahydrofuran und 1 ml Dimethylformamid gegeben. Das Reaktionsgemisch wird 150 Minuten unter Rückfluß zum Sieden erhitzt, dann auf 1,3 l Wasser gegossen und abgesaugt. Man erhält 36,7 g (92 % der Theorie) 2-Nitro-5-(2,6-dichlor-4-trifluormethyl-phenoxy)-benzylchlorid in Form gelber Kristalle vom Schmelzpunkt 84°C.

Beispiel 2

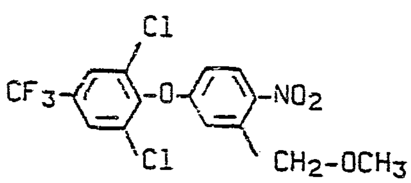

7,6 g (0,06 Mol) Dimethylsulfat werden bei 20°C zu einer Mischung aus 19,1 g (0,05 Mol) 2-Nitro-5-(2,6-dichlor-4-trifluormethyl-phenoxy)-benzylalkohol, 80 ml Diglykoldimethylether (Diglyme), 3,9 g.Kaliumhydroxid-Pulver und

10 ml Wasser gegeben. Das Reaktionsgemisch wird 2 Stunden bei 20°C gerührt und nach Zugabe von 1,2 g Dimethylsulfat und 0,6 g Kaliumhydroxid-Pulver weitere 3 Stunden gerührt, dann in 1 l Wasser gegossen und abgesaugt. Das Rohprodukt wird aus 60 ml Ligroin umkristallisiert. Man erhält 13,9 g (70 % der Theorie) (2-Nitro-5-(2,6-dichlor-4-trifluormethyl-phenoxy)-benzyl)-methylether in Form gelber Kristalle vom Schmelzpunkt 94°C.

Nach den in den Beispielen 1 und 2 beschriebenen Methoden werden auch die in der nachstehenden Tabelle 2 formelmäßig aufgeführten Verbindungen hergestellt:

Tabelle 2

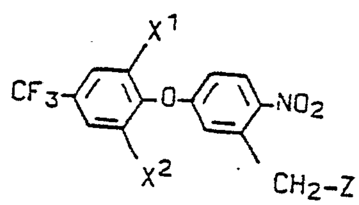

(I)

| Beispiel Nr. | $X^1$ | $X^2$ | Z | Schmelzpunkt °C; bzw. Brechungsindex |
|---|---|---|---|---|
| 3 | H | Cl | Cl | $n_D^{22}$ : 1,5690 |
| 4 | H | Cl | $OCH_3$ | 81 |

Beispiel 5

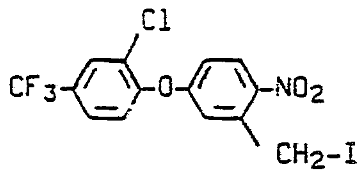

Eine Lösung von 109,8 g (0,3 Mol) 2-Nitro-5-(2-chlor-4-trifluormethyl-phenoxy)-benzylchlorid in 200 ml Aceton wird bei 20 bis 30°C zu einer Mischung aus 52,5 g (0,35 Mol) Natriumiodid und 200 ml Aceton gegeben. Das Reaktionsgemisch wird 4 Stunden unter Rückfluß zum Sieden erhitzt, dann eingeengt und mit Toluol und Wasser verrührt. Die organische Phase wird abgetrennt, nacheinander mit verdünnter wäßriger Natriumhydrogensulfitlösung und mit Wasser gewaschen, getrocknet, filtriert und eingeengt. Der Rückstand wird mit wenig kaltem Methanol digeriert und abgesaugt. Man erhält 104 g (76 % der Theorie) 2-Nitro-5-(2-chlor-4-trifluormethyl-phenoxy)-benzyliodid in Form blaßgelber Kristalle vom Schmelzpunkt 114°C.

Beispiel II-1

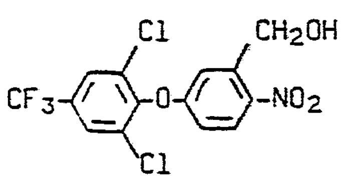

Eine Lösung von 41,5 g 5-(2,6-Dichlor-4-trifluormethyl-phenoxy)-2-nitrobenzoesäurechlorid in 100 ml Diglyme wird zu einem Gemisch aus 4,2 g Natriumboranat und 60 ml

Diglyme bei -5 bis -10°C gegeben. Das Reaktionsgemisch wird ca. 15 Stunden bei Raumtemperatur gerührt, mit 1,2 l Wasser verdünnt, mit Essigsäure angesäuert und abgesaugt. Man erhält 36 g (94 % der Theorie) 5-(2,6-Dichlor-4-trifluormethyl-phenoxy)-2-nitro-benzylalkohol vom Schmelzpunkt 143°C.

Beispiel II-2

Analog erhält man die Verbindung der Formel

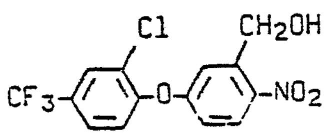

Schmelzpunkt 84°C.

In den folgenden Verwendungsbeispielen wird die nachstehend angegebene Verbindung als Vergleichssubstanz eingesetzt:

(A) =

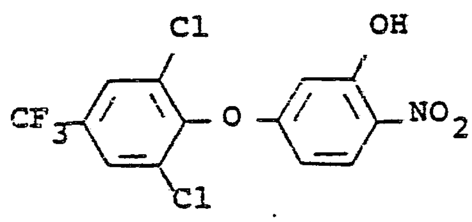

2-Nitro-5-(2,6-dichlor-4-trifluormethyl-phenoxy)-phenol
(bekannt aus US-PS 3 928 416).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % keine Wirkung (wie unbehandelte Kontrolle)
100 % totale Vernichtung

In diesem Test zeigen die erfindungsgemäßen Wirkstoffe (2) und (4) eine bessere selektive herbizide Wirksamkeit als die Vergleichssubstanz (A).

Beispiel B

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter in Vergleich zu den Kontrollpflanzen bonitiert. Es bedeuten:

- 0 kein Austrocknen der Blätter, kein Blattfall
- \+ leichtes Austrocknen der Blätter, geringer Blattfall
- ++ starkes Austrocknen der Blätter, starker Blattfall
- +++ sehr starkes Austrocknen der Blätter, sehr starker Blattfall

In diesem Test zeigen die erfindungsgemäßen Wirkstoffe (2) und (4) eine sehr gute Wirksamkeit.

Patentansprüche

1. 2-Nitro-5-(4-trifluormethyl-phenoxy)-benzyl-derivate der Formel

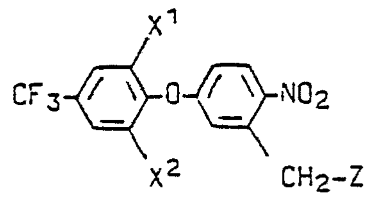

(I)

in welcher

$X^1$ und $X^2$ unabhängig voneinander für Wasserstoff oder Chlor stehen und

Z für Fluor, Chlor, Brom, Jod oder für den Rest -O-R steht, worin R für Methyl, Ethyl, Propyl, Butyl, Trifluormethyl, 2,2,2-Trifluorethyl, 2-Chlorethyl, 2-Methoxyethyl, 2-Ethoxyethyl, 2-Propoxyethyl, Methoxycarbonylmethyl, Methoxycarbonylethyl, Ethoxycarbonylmethyl, Ethoxycarbonylethyl, Dimethylaminocarbonylmethyl, Dimethylaminocarbonylethyl, Diethylaminocarbonylmethyl, Diethylaminocarbonylethyl, Methylpiperidinocarbonylmethyl, Methylpiperidinocarbonylethyl, Ethylpiperidinocarbonylmethyl, N-Methyl-N-phenyl-aminocarbonylmethyl oder N-Methyl-N-phenyl-aminocarbonylethyl steht.

2. Verfahren zur Herstellung von 2-Nitro-5-(4-trifluormethyl-phenoxy)-benzyl-derivaten der Formel

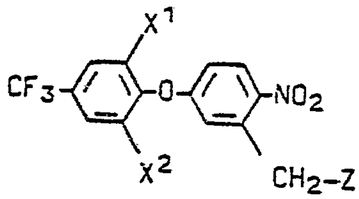

(I)

in welcher

$X^1$ und $X^2$ unabhängig voneinander für Wasserstoff oder Chlor stehen und

Z für Fluor, Chlor, Brom, Jod oder für den Rest -O-R steht, worin R für Methyl, Ethyl, Propyl, Butyl, Trifluormethyl, 2,2,2-Trifluorethyl, 2-Chlorethyl, 2-Methoxyethyl, 2-Ethoxyethyl, 2-Propoxyethyl, Methoxycarbonylmethyl, Methoxycarbonylethyl, Ethoxycarbonylmethyl, Ethoxycarbonylethyl, Dimethylaminocarbonylmethyl, Dimethylaminocarbonylethyl, Diethylaminocarbonylmethyl, Diethylaminocarbonylethyl, Methylpiperidinocarbonylmethyl, Methylpiperidinocarbonylethyl, Ethylpiperidinocarbonylmethyl, N-Methyl-N-phenyl-aminocarbonylmethyl oder N-Methyl-N-phenylaminocarbonylethyl steht,

dadurch gekennzeichnet, daß man

(a) 2-Nitro-5-(4-trifluormethyl-phenoxy)-benzylalkohole der Formel

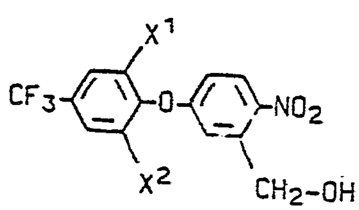

(II)

in welcher

$X^1$ und $X^2$ die oben angegebene Bedeutung haben,

α) mit zum Austausch von Hydroxy gegen Chlor oder Brom geeigneten Stoffen, gegebenenfalls in Gegenwart eines Katalysators und gegebenenfalls in Gegenwart eines Verdünnungsmittels umsetzt,

oder

ß) mit Alkylierungsmitteln der Formel

$$Z^1 - R \qquad (III)$$

in welcher

$Z^1$ für Chlor, Brom oder Jod steht und

R die oben angegebene Bedeutung hat,

gegebenenfalls in Gegenwart eines Säureakzeptors und gegebenenfalls in Gegenwart eines Verdünnungsmittels umsetzt,

oder

γ) mit Dimethylsulfat oder Diethylsulfat gegebenenfalls in Gegenwart eines Säureakzeptors und gegebenenfalls in Gegenwart eines Verdünnungsmittels umsetzt,

oder

b) 2-Nitro-5-(4-trifluormethyl-phenoxy)-benzyl-halogenide der Formel

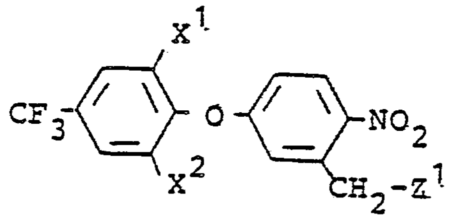

(Ia)

in welcher

$X^1$, $X^2$ und $Z^1$ die oben angegebene Bedeutung haben,

α) mit Fluorierungsmitteln oder, sofern $Z^1$ nicht für Jod steht, mit Alkaliiodiden gegebenenfalls in Gegenwart eines Katalysators sowie gegebenenfalls in Gegenwart eines Verdünnungsmittels umsetzt,

oder

ß) mit Verbindungen der Formel

$$R - O - X \qquad (IV)$$

in welcher

R die oben angegebene Bedeutung hat und

X für Wasserstoff, Lithium, Natrium oder Kalium steht,

gegebenenfalls in Gegenwart eines Säureakzeptors sowie gegebenenfalls in Gegenwart eines Verdünnungsmittels umsetzt,

oder

c) 3-(4-Trifluormethyl-phenoxy)-benzylderivate der Formel

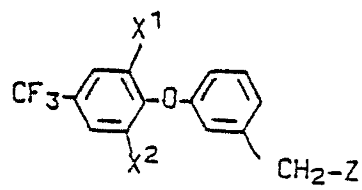

(V)

in welcher

$X^1$,$X^2$ und Z die oben angegebenen Bedeutungen haben,

mit Salpetersäure gegebenenfalls in Gegenwart eines Katalysators und gegebenenfalls in Gegenwart eines Verdünnungsmittels umsetzt.

3. Herbizide und pflanzenwuchsregulierende Mittel, gekennzeichnet durch einen Gehalt an mindestens

einem 2-Nitro-5-(4-trifluormethyl-phenoxy)-benzylderivat der Formel (I).

4. Verfahren zur Bekämpfung von Unkräutern bzw. zur Regulierung des Pflanzenwachstums, dadurch gekennzeichnet, daß man 2-Nitro-5-(4-trifluormethyl-phenoxy)-benzylderivate der Formel (I) auf die Pflanzen und/oder deren Lebensraum ausbringt.

5. Verwendung von 2-Nitro-5-(4-trifluormethyl-phenoxy)-benzylderivaten der Formel (I) zur Bekämpfung von Unkräutern bzw. zur Regulierung des Pflanzenwachstums.

6. Verfahren zur Herstellung von herbiziden bzw. pflanzenwachstumsregulierenden Mitteln, dadurch gekennzeichnet, daß man 2-Nitro-5-(4-trifluormethyl-phenoxy)-benzylderivate der Formel (I) mit Streckmitteln und/oder oberflächenaktiven Stoffen vermischt.

7. 2-Nitro-5-(4-trifluormethyl-phenoxy)-benzylderivat der Formel

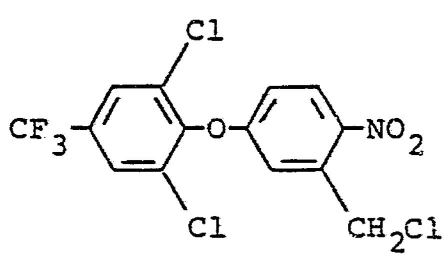

8. 2-Nitro-5-(4-trifluormethyl-phenoxy)-benzylderivat der Formel

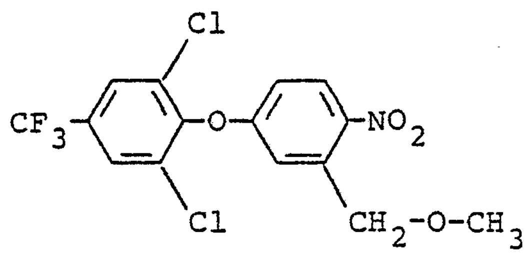

9. 2-Nitro-5-(4-trifluormethyl-phenoxy)-benzylderivat der Formel

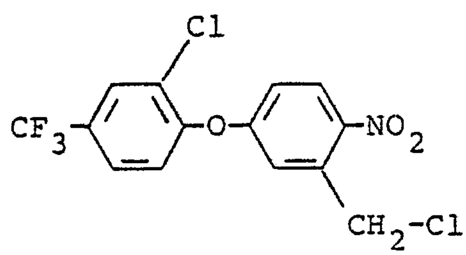

10. 2-Nitro-5-(4-trifluormethyl-phenoxy)-benzylderivat der Formel

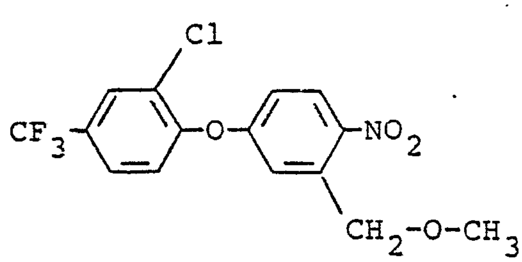

Europäisches Patentamt

# EUROPÄISCHER RECHERCHENBERICHT

0077963

Nummer der Anmeldung

EP 82 10 9385

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| P,X | EP-A-0 056 119 (BASF)<br>* Zusammenfassung; Beispiele 3,9,12; Seiten 28-30, Verbindung Nr. 87,95,109,120,127; Seiten 46,47 * | 1-6,9, 10 | C 07 C 79/355<br>C 07 C 76/02<br>A 01 N 33/22 |
| A | GB-A-2 060 634 (CHEVRON RESEARCH)<br>* Zusammenfassung; Seite 3, Zeilen 38-41 * | 3-6 | |
| A | US-A-4 093 446 (H.O. BAYER)<br>* Spalte 1, Zeile 48 - Spalte 2, Zeile 27; Spalte 5, Zeilen 42,43; Spalte 6, Zeilen 24-31 * | 3-6 | |
| | | | RECHERCHIERTE SACHGEBIETE (Int. Cl. 3) |
| | | | C 07 C 76/00<br>C 07 C 79/00 |

Der vorliegende Recherchenbericht wurde für alle Patentansprüche erstellt.

| Recherchenort | Abschlußdatum der Recherche | Prüfer |
|---|---|---|
| DEN HAAG | 18-01-1983 | WRIGHT M.W. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82